(19) Europäisches Patentamt
European Patent Office
Office européen des brevets

(11) Veröffentlichungsnummer: **0 193 807**
**A2**

# (12) EUROPÄISCHE PATENTANMELDUNG

(21) Anmeldenummer: **86102255.6**

(22) Anmeldetag: **21.02.86**

(51) Int. Cl.[4]: **C 07 D 251/16**

(30) Priorität: **05.03.85 DE 3507750**

(43) Veröffentlichungstag der Anmeldung: **10.09.86**
**Patentblatt 86/37**

(84) Benannte Vertragsstaaten: **AT BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Fauss, Rudolf, Dr., Gerstenkamp 10,**
**D-5000 Köln 80 (DE)**
Erfinder: **Riebel, Hans-Jochem, Dr., In der Beek 92,**
**D-5600 Wuppertal 1 (DE)**

## (54) Verfahren zur Herstellung von 4-Alkoxy-6-alkyl-2-cyanamino-1,3,5-triazinen.

(57) Man erhält nach einem neuartigen Verfahren in guten Ausbeuten 4-Alkoxy-6-alkyl-2-cyanamino-1,3,5-triazine der allgemeinen Formel (I)

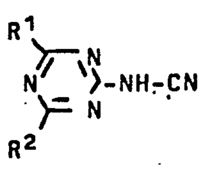

(I)

in welcher
$R^1$ für Alkyl steht und
$R^2$ für Alkoxy steht,
(welche als Zwischenprodukte zur Herstellung bekannter, herbizid wirksamer Guanidin-Derivate verwendet werden können), indem man die bekannten 4-Alkoxy-6-alkyl-2-amino-1,3,5-triazine der Formel (II)

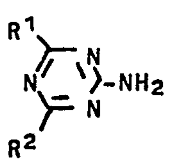

(II)

mit Halogencyanen der Formel (III)

Hal–CN (III)

in welcher
Hal für Halogen steht,
in Gegenwart der zweifach molaren Menge Butyllithium und in Gegenwart von Verdünnungsmitteln umsetzt und anschließend mit Wasser versetzt und ansäuert.

EP 0 193 807 A2

BAYER AKTIENGESELLSCHAFT    5090 Leverkusen-Bayerwerk

Konzernverwaltung RP    Bi-mü /Ma
Patentabteilung    IVa/ZP

Verfahren zur Herstellung von 4-Alkoxy-6-alkyl-2-cyanamino-1,3,5-triazinen

Die vorliegende Erfindung betrifft ein neuartiges Verfahren zur Herstellung von 4-Alkoxy-6-alkyl-2-cyanamino-1,3,5-triazinen, welche als Zwischenprodukte für die Herstellung von Herbiziden und Pflanzenwachstumsregulatoren verwendet werden können.

Es ist bereits bekannt, daß 2-Cyanamino-1,3,5-triazine durch Umsetzung von Alkalimetall- oder Erdalkalimetall-Salzen von Cyanamid mit den entsprechenden 2-Halogen-1,3,5-triazinen erhalten werden (vgl. z.B. DE-OS 33 34 455 / EP-A-121 082). Dieses Verfahren ist jedoch wegen unbefriedigender Herstellungsmethoden für die benötigten Ausgangsprodukte nur sehr begrenzt anwendbar. Es besteht daher ein Bedarf an einem neuen, breit anwendbaren Herstellungsverfahren für 4-Alkoxy-6-alkyl-2-cyanamino-1,3,5-triazine.

Es wurde nun gefunden, daß man 4-Alkoxy-6-alkyl-2-cyanamino-1,3,5-triazine der allgemeinen Formel (I)

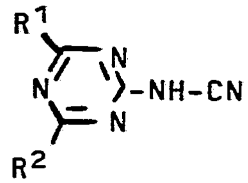 (I)

in welcher

$R^1$ für Alkyl steht und

$R^2$ für Alkoxy steht,

erhält, wenn man 4-Alkoxy-6-alkyl-2-amino-1,3,5-triazine der Formel (II)

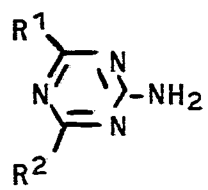 (II)

in welcher

$R^1$ und $R^2$ die oben angegebenen Bedeutungen haben,

mit Halogencyanen der Formel (III)

Hal-CN (III)

in welcher

Hal für Halogen steht,

in Gegenwart der zweifachen molaren Menge Butyllithium und in Gegenwart von Verdünnungsmitteln umsetzt und anschließend mit Wasser versetzt und ansäuert.

Ueberraschenderweise gelingt es mit diesem neuartigen Verfahren, die Verbindungen der Formel (I) in guten Ausbeuten zu erhalten. Bei der Herstellung der Verbindungen der Formel (I) nach dem Stand der Technik aus Alkalimetall- bzw. Erdalkalimetall-Salze von Cyanamid und den entsprechenden Halogen-1,3,5-triazinen sind die Ausbeuten sehr unbefriedigend. Es war auch nicht vorhersehbar, daß pro Mol der Ausgangsstoffe (II) und (III) 2 Mol Butyllithium eingesetzt werden müssen; die Verwendung von 1 Mol Butyllithium führt nur zu 50 % Umsatz.

Bevorzugt werden mit Hilfe des erfindungsgemäßen Verfahrens die Verbindungen der Formel (I) hergestellt, in welcher

$R^1$ für Alkyl mit 1 bis 6 Kohlenstoffatomen steht und

$R^2$ für Alkoxy mit 1 bis 6 Kohlenstoffatomen steht.

Besonders bevorzugt werden diejenigen Verbindungen der Formel (I) hergestellt, in welcher

$R^1$ für Methyl, Ethyl, n-Propyl, i-Propyl, n-Butyl, i-Butyl, sec. Butyl oder tert. Butyl steht und

$R^2$ für Methoxy, Ethoxy, n-Propoxy, i-Propoxy, n-Butoxy, i-Butoxy, sec. Butoxy oder tert. Butoxy steht.

Ganz besonders bevorzugt werden diejenigen Verbindungen der Formel (I) hergestellt, in welcher

$R^1$ für Methyl, Ethyl, n-Propyl, i-Propyl oder n-Butyl steht und

$R^2$ für Methoxy, Ethoxy, n-Propoxy, i-Propoxy oder n-Butoxy steht.

Verwendet man beispielsweise für das erfindungsgemäße Verfahren 2-Amino-4-methoxy-6-methyl-1,3,5-triazin und Chlorcyan als Ausgangsstoffe, so kann die Reaktion durch das folgende Formelschema wiedergegeben werden:

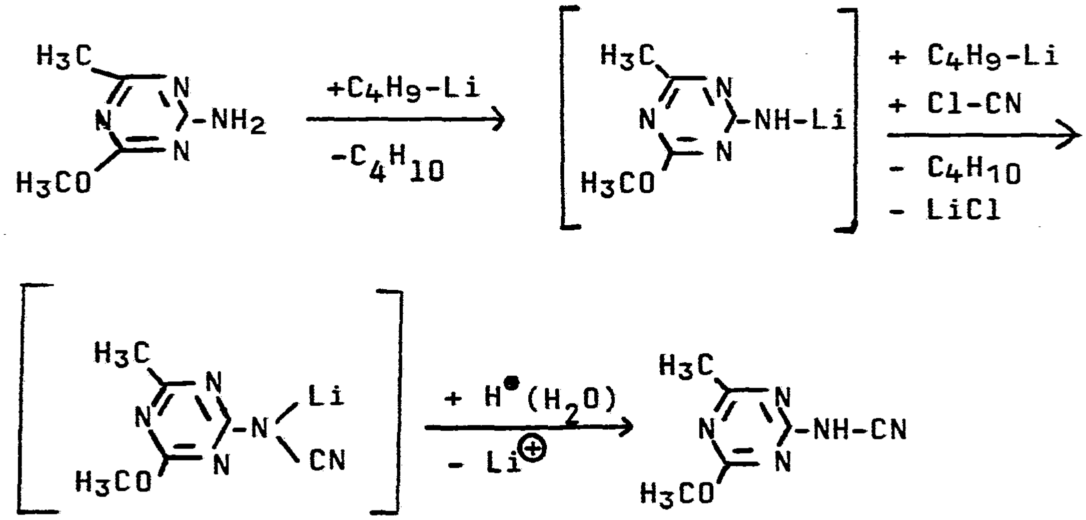


Die als Ausgangsstoffe für das erfinderische Verfahren zu verwendenden 4-Alkoxy-6-alkyl-2-amino-1,3,5-triazine sind durch die Formel (II) allgemein definiert. In dieser Formel stehen $R^1$ und $R^2$ bevorzugt für diejenigen Reste, welche oben im Rahmen der Substituentendefinition der Formel (I) vorzugsweise bzw. als insbesondere bevorzugt angegeben sind.

Als Beispiele für die Verbindungen der Formel (II) seien genannt:

6-Methyl-4-methoxy-, 6-Methyl-4-ethoxy-, 6-Methyl-4-n-propoxy-, 6-Methyl-4-i-propoxy-, 6-Methyl-4-n-butoxy-, 6-Methyl-4-i-butoxy-, 6-Methyl-4-sec.-butoxy-, 6-Methyl-4-tert.butoxy-, 6-Ethyl-4-methoxy-, 6-Ethyl-4-ethoxy-, 6-Ethyl-4-n-propoxy-, 6-Ethyl-4-i-propoxy-, 6-Ethyl-4-n-butoxy-, 6-Ethyl-4-i-butoxy-, 6-Ethyl-4-sec.-butoxy-, 6-Ethyl-4-tert-butoxy-, 6-n-Propyl-4-methoxy-, 6-n-Propyl-4-ethoxy-, 6-n-Propyl-4-n-propoxy-, 6-n-Propyl-4-i-propoxy-, 6-n-Propyl-4-n-butoxy-, 6-n-Propyl-4-i-butoxy-, 6-n-Propyl-4-sec.-butoxy-, 6-n-Propyl-4-tert.-butoxy-, 6-i-Propyl-4-methoxy-, 6-i-Propyl-4-ethoxy-, 6-i-Propyl-4-n-propoxy-, 6-i-Propyl-4-i-propoxy-, 6-i-Propyl-4-n-butoxy-, 6-i-Propyl-4-i-butoxy-, 6-i-Propyl-4-sec.-butoxy-, 6-i-Propyl-4-tert.-butoxy-, 6-n-Butyl-4-methoxy-, 6-n-Butyl-4-ethoxy-, 6-n-Butyl-4-n-propoxy-, 6-n-Butyl-4-i-propoxy-, 6-n-Butyl-4-n-butoxy-, 6-n-Butyl-4-i-butoxy-, 6-n-Butyl-4-sec.-butoxy-, 6-n-Butyl-4-tert.-butoxy-, 6-i-Butyl-4-methoxy-, 6-i-Butyl-4-ethoxy-, 6-i-Butyl-4-n-propoxy-, 6-i-Butyl-4-i-propoxy-, 6-i-Butyl-4-n-butoxy-, 6-i-Butyl-4-i-butoxy-, 6-i-Butyl-4-sec.-butoxy-, 6-i-Butyl-4-tert.-butoxy-, 6-sec.-Butyl-4-methoxy-, 6-sec.-Butyl-4-ethoxy-, 6-sec.-Butyl-4-n-propoxy-, 6-sec.-Butyl-4-i-propoxy-, 6-sec.-Butyl-4-n-butoxy-, 6-sec.-Butyl-4-i-butoxy-, 6-sec.-Butyl-4-sec.-butoxy-, 6-sec.-Butyl-4-tert.-butoxy-, 6-tert.-Butyl-4-methoxy-, 6-tert.-Butyl-4-ethoxy-, 6-tert.-Butyl-4-n-propoxy-, 6-tert.-Butyl-4-i-propoxy-, 6-tert.-Butyl-4-n-butoxy-, 6-tert.-Butyl-4-i-butoxy-, 6-tert.-Butyl-4-sec.-butoxy- und 6-tert.-Butyl-4-tert.-butoxy-2-amino-1,3,5-triazin.

Die Verbindungen der Formel (II) sind bekannte Verbindungen der organischen Chemie.

Die außerdem als Ausgangsstoffe für das erfindungsgemäße Verfahren zu verwendenden Halogencyane sind durch die Formel (III) allgemein definiert. In dieser Formel steht Hal für Halogen, vorzugsweise für Chlor oder Brom.

Als Beispiele für die Verbindungen der Formel (III) seien genannt:

Chlorcyan und Bromcyan.

Die Verbindungen der Formel (III) sind allgemein bekannte Verbindungen der organischen Chemie.

Das erfindungsgemäße Verfahren wird in Gegenwart von wasserfreien, inerten Verdünnungsmitteln durchgeführt. Hierzu gehören insbesondere aliphatische Kohlenwasserstoffe wie Hexan, Ether wie Diethyl- und Dibutylether, Glykoldimethylether und Diglykoldimethylether, Tetrahydrofuran und Dioxan, Amide, wie z.B. Dimethylformamid, Dimethylacetamid und N-Methylpyrrolidon, sowie Dimethylsulfoxid, Tetramethylensulfon und Hexamethylphosphorsäuretriamid.

Das erfindungsgemäße Verfahren wird im allgemeinen bei Temperaturen zwischen -20°C und +60°C durchgeführt. Bevorzugt wird der Bereich zwischen -10°C und +40°C. Die Umsetzungen werden im allgemeinen bei Normaldruck durchgeführt.

Bei der Durchführung des erfindungsgemäßen Verfahrens setzt man auf 1 Mol der Verbindung der Formel (II) insgesamt 2 Mol Butyllithium und 1 Mol Halogencyan der Formel (III) ein und säuert anschließend mit einer Säure wie z.B. Salzsäure oder Essigsäure an.
Bei der Reaktionsdurchführung geht man im allgemeinen so vor, daß ein Gemisch aus Verdünnungsmittel und eine Verbindung der Formel (II) mit einer äquimolaren Menge Butyllithium versetzt wird und nach Abklingen der exothermen Reaktion gleichzeitig eine äquimolare Menge Butyllithium und eine Lösung aus Verdünnungsmittel und eine äquimolare Menge Halogencyan der Formel (III) zudosiert wird.
Nach der Reaktion wird das Lösungsmittel entfernt, der Rückstand mit Wasser versetzt, vom ungelösten Feststoff befreit und angesäuert. Die entstehenden kristallinen Verbindungen der Formel (I) werden abgesaugt, gegebenenfalls umkristallisiert und anschließend getrocknet.

Die nach dem erfindungsgemäßen Verfahren herzustellenden 4-Alkoxy-6-alkyl-2-cyanamino-1,3,5-triazine können als Zwischenprodukte für die Herstellung von Guanidin-Derivaten, die als Herbizide und Pflanzenwuchsregulatoren wirksam sind, eingesetzt werden (vgl. EP-OS 121 082).

Herstellungsbeispiele

Beispiel 1

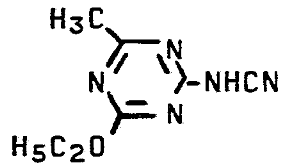

Zu einer Suspension aus 17,7 g (0,115 Mol) 2-Amino-4-ethoxy-6-methyl-1,3,5-triazin und 200 ml Tetrahydrofuran werden bei 20°C 50 ml einer ca. 23-%igen Lösung von Butyllithium (0,115 Mol) in Hexan getropft. Nach Abklingen der exothermen Reaktion werden zum Reaktionsgemisch bei 15-20°C gleichzeitig 50 ml der ca. 23-%igen Lösung von Butyllithium (0,115 Mol) in Hexan und eine Lösung von 5,9 ml (0,115 Mol) Chlorcyan in 45 ml Tetrahydrofuran getropft und bei ca. 20°C nachgerührt. Anschließend wird eingeengt und mit kaltem Wasser versetzt. Der Rückstand wird entfernt und das Filtrat angesäuert (pH-Wert $<$4). Die entstehenden Kristalle werden abgesaugt und getrocknet.

Man erhält 13,6 g (66 % der Theorie) 2-Cyanamino-4-ethoxy-6-methyl-1,3,5-triazin vom Schmelzpunkt Fp: 192-194°C (Zers.).

Analog Beispiel 1 wird die folgende Verbindung der Formel (I) hergestellt:

Beispiel 2

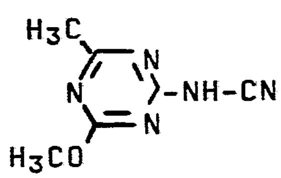

Fp.: 184°C (Zers.)

Patentansprüche

1) Verfahren zur Herstellung von 4-Alkoxy-6-alkyl-2-cyanamino-1,3,5-triazinen der allgemeinen Formel (I),

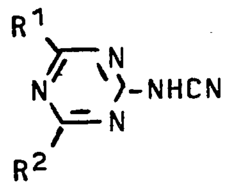

(I)

in welcher

$R^1$ für Alkyl steht und

$R^2$ für Alkoxy steht,

dadurch gekennzeichnet, daß man 4-Alkoxy-6-alkyl-2-amino-1,3,5-triazine der Formel (II),

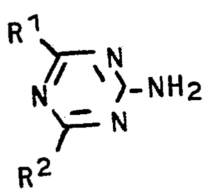

(II)

in welcher

$R^1$ und $R^2$ die oben angegebenen Bedeutungen haben,

mit Halogencyanen der Formel (III)

Hal-CN (III)

in welcher

Hal für Halogen steht,

in Gegenwart der zweifachen molaren Menge Butyllithium und in Gegenwart von Verdünnungsmitteln umsetzt und anschließend mit Wasser versetzt und ansäuert.